(19) **Europäisches Patentamt**
**European Patent Office**
**Office européen des brevets**

(11) **EP 2 814 572 B1**

(12) **EUROPÄISCHE PATENTSCHRIFT**

(45) Veröffentlichungstag und Bekanntmachung des Hinweises auf die Patenterteilung:
**19.02.2020 Patentblatt 2020/08**

(21) Anmeldenummer: **13714605.6**

(22) Anmeldetag: **28.03.2013**

(51) Int Cl.:
**A61N 5/10** *(2006.01)*

(86) Internationale Anmeldenummer:
**PCT/EP2013/056644**

(87) Internationale Veröffentlichungsnummer:
**WO 2013/170996 (21.11.2013 Gazette 2013/47)**

(54) **VERFAHREN UND VORRICHTUNG ZUM BESTIMMEN EINES BESTRAHLUNGSPLANS FÜR EINE PARTIKELBESTRAHLUNGSANLAGE**

METHOD AND DEVICE FOR DETERMINING AN IRRADIATION PLAN FOR A PARTICLE IRRADIATION UNIT

PROCÉDÉ ET DISPOSITIF DE DÉFINITION D'UN PLAN D'IRRADIATION POUR UNE INSTALLATION D'IRRADIATION DE PARTICULES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.05.2012 DE 102012208027**

(43) Veröffentlichungstag der Anmeldung:
**24.12.2014 Patentblatt 2014/52**

(73) Patentinhaber: **Siemens Healthcare GmbH 91052 Erlangen (DE)**

(72) Erfinder:
• ELSÄSSER, Thilo
  91054 Buckenhof (DE)
• GEMMEL, Alexander
  91056 Erlangen (DE)
• HANSMANN, Thomas
  69121 Heidelberg (DE)
• RIETZEL, Eike
  64331 Weiterstadt (DE)

(56) Entgegenhaltungen:
**EP-A1- 2 221 088    WO-A2-2008/003526**

EP 2 814 572 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung ist in den Ansprüche definiert. Die Erfindung betrifft ein Verfahren und eine Vorrichtung zur Bestimmung eines Bestrahlungsplans für eine Partikelbestrahlungsanlage sowie eine entsprechend ausgestaltete Partikelbestrahlungsanlage.

Beispielsweise im Rahmen einer Therapieplanung bei der Partikeltherapie wird im Vorfeld ein Bestrahlungsplan erstellt, welcher Steuerparameter zur Bestrahlung eines Untersuchungsobjekts definiert. Mit dem Bestrahlungsplan wird die Bestrahlung eines Gegenstands gemäß bestimmten Vorgaben (z.B. Zielvolumen und Dosisverteilung) geplant.

Die Partikeltherapie ist ein mittlerweile etabliertes Verfahren, mit welchem insbesondere von Tumorerkrankungen befallenes Gewebe bestrahlt wird. Bei der Partikeltherapie werden geladene Partikel, wie beispielsweise Protonen oder Kohlenstoff-Ionen oder andere Ionen auf hohe Energien beschleunigt, zu einem Partikelstrahl geformt und über ein Hochenergiestrahl-Transportsystem zu einem (oder mehreren) Bestrahlungsräumen geführt. In einem Bestrahlungsraum wird das Zielvolumen des Behandlungsobjekts mit dem Partikelstrahl bestrahlt, wobei zwangsweise auch Gewebe außerhalb des Zielvolumens bestrahlt wird.

Bei der Partikeltherapie mit aktivem Scanverfahren werden einzelne Rasterpunkte mit Partikelstrahlen unterschiedlicher Intensität (Teilchenzahl pro Zeiteinheit), wobei die unterschiedlichen Intensitäten mehrere Größenordnungen überdecken können. Zur Ortsmessung und Intensitätsmessung mittels Ionisationskammern wird dabei ein Monitoringsystem der Partikelbestrahlungsanlage eingesetzt.

Unter einem Rasterpunkt ist dabei kein mathematischer Punkt im Zielvolumen zu verstehen. Stattdessen definiert der Rasterpunkt eine kleine Fläche oder Schicht meist im Zielvolumen, welche orthogonal zum Teilchenstrahl steht. Der Teilchenstrahl durchläuft somit den Rasterpunkt bzw. die von ihm definierte Schicht und deponiert entlang seiner Bahn die zu applizierende Dosis, wobei der größte Anteil dieser Dosis im so genannten "Bragg Peak" appliziert wird bzw. appliziert werden sollte.

Die vorliegende Erfindung stellt sich die Aufgabe, den Patientendurchsatz bei einer Partikeltherapie mit aktiver Energievariation im Vergleich zum Stand der Technik zu verkürzen.

Erfindungsgemäß wird diese Aufgabe durch ein Verfahren zum Bestimmen eines Bestrahlungsplans nach Anspruch 1, durch eine Vorrichtung zum Bestimmen eines Bestrahlungsplans nach Anspruch 14, durch eine Partikelbestrahlungsanlage nach Anspruch 16, durch ein Computerprogrammprodukt nach Anspruch 17 und durch einen elektronisch lesbaren Datenträger nach Anspruch 18 gelöst. Die abhängigen Ansprüche definieren bevorzugte und vorteilhafte Ausführungsformen der vorliegenden Erfindung.

Im Rahmen der vorliegenden Erfindung wird ein Verfahren zum (insbesondere automatischen) Bestimmen eines Bestrahlungsplans für eine Partikelbestrahlungsanlage bereitgestellt. Dabei wird mit Hilfe der Partikelbestrahlungsanlage gemäß dem Bestrahlungsplan ein Zielvolumen innerhalb eines Untersuchungsobjekts mit einem Partikelstrahl bzw. Teilchenstrahl bestrahlt. Der Bestrahlungsplan wird dabei ausgehend von einem vorgegebenen Zielvolumen und einer vorbestimmten Dosisverteilung (Soll-Dosisverteilung) innerhalb dieses Zielvolumens bestimmt, um anhand des Bestrahlungsplans die Dosis des Teilchenstrahls mit einer hohen Qualität (z.B. möglichst exakt entsprechend der vorbestimmten Dosisverteilung innerhalb des Zielvolumens) zu deponieren oder zu applizieren. Darüber hinaus wird erfindungsgemäß bei der Bestimmung des Bestrahlungsplans eine Bestrahlungsdauer berücksichtigt, so dass der Bestrahlungsplan derart bestimmt wird, dass die Bestrahlungsdauer möglichst kurz ist.

Durch die Berücksichtigung der Bestrahlungsdauer bei der Erstellung des Bestrahlungsplans kann beispielsweise die Dauer, während welcher ein Patient im Rahmen der Partikeltherapie bestrahlt wird, verkürzt werden, was wiederum einen positiven Einfluss auf den Patientendurchsatz hat.

Der Bestrahlungsplan kann beispielsweise mit Hilfe eines Optimierungsverfahrens bestimmt werden, bei welchem eine Maßzahl unter anderem abhängig von der Qualität der Dosisverteilung gemäß Bestrahlungsplan und von der Bestrahlungsdauer berechnet wird. Das Optimierungsverfahren bestimmt dabei denjenigen Bestrahlungsplan, bei welchem die Maßzahl den besten Wert aufweist, wobei die Maßzahl umso besser ist, je besser die Qualität der Dosisverteilung gemäß Bestrahlungsplan und je kürzer die Bestrahlungsdauer ist.

Die Qualität der Dosisverteilung und die Kürze der Bestrahlungsdauer sind im Allgemeinen zwei gegenläufige Anforderungen an den Bestrahlungsplan. Die Kostenfunktion oder Optimierungsfunktion, mit welcher das Optimierungsverfahren die Maßzahl bestimmt, umfasst daher mindestens einen Term zur Bewertung der Qualität der Dosisverteilung und zumindest einen Term zur Bewertung der Bestrahlungsdauer. Der oder die Terme zur Bewertung der Qualität der Dosisverteilung ergeben eine umso bessere Maßzahl, je mehr die Dosisverteilung gemäß Bestrahlungsplan der vorgegebenen Soll-Dosisverteilung entspricht. Die Qualität der Dosisverteilung (d.h. die Qualität, mit welcher die Dosis des Teilchenstrahls anhand des Bestrahlungsplans appliziert wird) kann dabei beispielsweise an der Einhaltung bestimmter Toleranzgrenzen von Risikoorganen, anhand von Dosis-Volumen-Histogrammen, anhand der Einhaltung von Toleranzgrenzen hinsichtlich der Dosisverteilung innerhalb des Zielvolumens ("dosis constraints of planning target volume") und/oder anhand der Übereinstimmung zwischen tatsächlicher Dosisverteilung und Soll-Dosisverteilung (insbesondere müssen die absoluten Werte der tatsächlichen Dosisverteilung (z.B. gemäß Bestrahlungsplan) den absoluten Werten der Soll-Dosisverteilung entsprechen) bestimmt werden. Diese Qualitätsdefinition gilt für die gesamte Erfindung.

Bei der Bestrahlungsdauer kann es sich erfindungsgemäß um eine Gesamtbestrahlungsdauer (gesamte zeitliche Dauer der Bestrahlung des Untersuchungsobjekts mit dem Teilchenstrahl) oder um eine Bestrahlungsdauer einer bestimmten Isoenergieschicht des Zielvolumens handeln.

Wenn zur Bestrahlung derselben Isoenergieschicht ein Spill (Beschleunigerzyklus) zusätzlich benötigt wird, steigt die Bestrahlungsdauer dieser Isoenergieschicht sprunghaft an, da die Erzeugung eines neuen Spills ca. 4 bis 5 s dauert. Daher führt die Bestrahlungsdauer der Isoenergieschicht als Optimierungskriterium dazu, dass neben der Optimierung der Summe aller Teilchenzahlen für diese Isoenergieschicht im Zusammenspiel mit der für die Isoenergieschicht eingestellten Intensitätsstufe die Rasterpunkte der Isoenergieschicht möglichst mit einer geringen Anzahl von Spills (insbesondere mit nur einem Spill) bestrahlt werden.

Dabei wird die Gesamtbestrahlungsdauer T durch die folgende Gleichung (1) bestimmt, wobei numSp der Anzahl der Spills entspricht, wobei TBschl derjenigen Zeitspanne (ca. 4 - 5 s) entspricht, welche die Partikel eines neuen Spills benötigen, um auf die angeforderte Geschwindigkeit beschleunigt zu werden, und wobei $TSp_i$ der Zeitdauer entspricht, während welcher der i-te-Spill in das Zielvolumen gestrahlt wird.

$$T = numSp \times TBschl + \sum_{i=1}^{numSp} TSp_i \qquad (1).$$

Die Zeitdauer $TSp_i$ wird mit Hilfe der Gleichung (2) berechnet, wobei $numPartSp_i$ der Anzahl der Teilchen bzw. Partikel im i-ten Spill entspricht, wobei $nomInt_i$ der nominalen Intensität des i-ten Spills entspricht und wobei EffF dem Effizienzfaktor der Partikelbestrahlungsanlage entspricht.

$$TSp_i = \frac{numPartSp_i}{nomInt_i \times EffF} \qquad (2).$$

Man erkennt an der Gleichung (1), dass die Anzahl (numSp) der Spills die Gesamtbestrahlungsdauer stark beeinflusst. Daher wird das mit Gleichung (1) arbeitende erfindungsgemäße Verfahren zum einen versuchen, möglichst nur einen Spill pro Isoenergieschicht einzusetzen, und zum anderen versuchen, die Anzahl der bestrahlten Isoenergieschichten möglichst klein zu halten.

Eine weitere Variante zur Berechnung der Bestrahlungsdauer $TSp_i$ des i-ten Spills ist in der folgenden Gleichung (3) angegeben.

$$TSp_i = \sum_{j=1}^{numSpot_i} \frac{numPartSpot_{j,i}}{Int_{j,i}} \qquad (3)$$

Dabei entspricht $numSpot_i$ der Anzahl der Rasterpunkte, welche von dem i-ten Spill bestrahlt werden, und $numPartSpot_{j,i}$ entspricht der Anzahl der Teilchen, welche von dem i-ten Spill beim j-ten Rasterpunkt von dem i-ten Spill appliziert werden. $Int_{j,i}$ gibt die Intensität an, mit welcher der j-te Rasterpunkt von dem i-ten Spill bestrahlt wird. Diese Intensität $Int_{j,i}$ wird wiederum durch die folgende Gleichung (4) berechnet.

$$Int_{j,i} = m \times \sum_{k=1}^{j} numPartSpot_{k,i} + b \qquad (4)$$

Dabei entspricht m einer Steigung einer Geraden und b einem y-Achsenabschnitt dieser Geraden. Die Gerade beschreibt einen empirisch ermittelten Zusammenhang zwischen der Intensität eines Spills in Abhängigkeit von der Partikelanzahl.

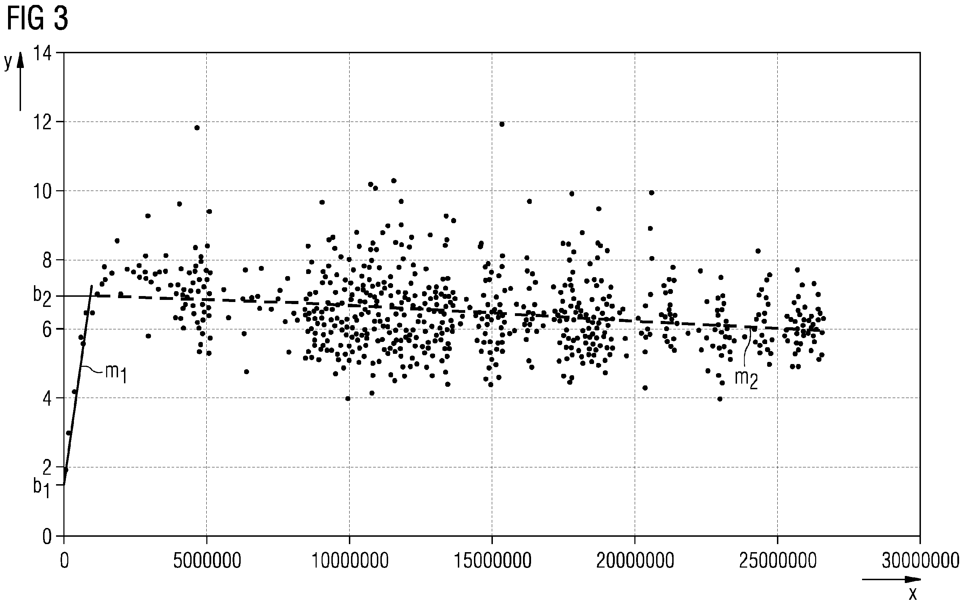

Wie später noch mit Fig. 3 im Detail erläutert werden wird, berücksichtigt die Berechnung der Bestrahlungsdauer eines Spills mit den Gleichungen (3) und (4), dass die Intensität während eines Spills nicht konstant ist, sondern z.B. linear mit der Partikelanzahl abfallen kann. Diese Partikelanzahl entspricht dabei der Anzahl der Partikel bzw. Teilchen, welche von Beginn an von dem i-ten Spill bis einschließlich der Bestrahlung des j-ten Rasterpunkts, bezüglich dessen Bestrahlung die Intensität bestimmt wird, abgegeben worden sind. Mit anderen Worten entspricht diese Partikelanzahl der Summe $\sum_{k=1}^{j} numPartSpot_{k,j}$ .

Je exakter die Bestrahlungsdauer eines Spills berechnet wird, desto genauer lassen sich Auswirkungen auf die Bestrahlungsdauer eines Spills und damit auf die Gesamtbestrahlungsdauer abschätzen. Dadurch kann vorteilhafterweise der Bestrahlungsplan im Vergleich zum Stand der Technik besser (insbesondere bezüglich der Gesamtbestrahlungsdauer) optimiert werden.

Gemäß einer bevorzugten erfindungsgemäßen Ausführungsform werden bei der Bestimmung der Intensität in Abhängigkeit von der Partikelanzahl zwei unterschiedliche Geraden, z.B. ein erster aufsteigender Ast und ein zweiter absteigender Ast, unterschieden. Anders ausgedrückt besitzt sowohl die Steigung m als auch der y-Achsenabschnitt abhängig davon einen anderen Wert, ob die Partikelanzahl kleiner oder größer als ein empirisch ermittelter Partikelanzahlschwellenwert (siehe Bezugszeichen 31 in Fig. 3) ist.

Zur Bestimmung der Steigung m und des y-Achsenabschnitts sowohl des aufsteigenden als auch des absteigenden Astes kann eine beliebige Optimierungsmethode (z.B. Methode der kleinsten Quadrate) eingesetzt werden, um ausgehend von beispielhaft erfassten Messpunkten (Intensität über Partikelanzahl) die Parameter (Steigung, y-Achsenabschnitt) zu bestimmen. Dabei ist insbesondere zu berücksichtigen, dass der y-Achsenabschnitt des abfallenden Astes von dem Effizienzfaktor der Partikelbestrahlungsanlage abhängt.

Erfindungsgemäß ist es möglich, dass der Bestrahlungsplan abhängig von dem Effizienzfaktor der Partikelbestrahlungsanlage bestimmt wird. Dabei entspricht der Effizienzfaktor dem durchschnittlichen Verhältnis der tatsächlichen bzw. gemessenen Intensität zur z.B. gemäß Bestrahlungsplan vorgegebenen Intensität.

Indem der Effizienzfaktor bei der Erstellung des Bestrahlungsplans berücksichtigt wird, kann vorteilhafterweise beispielsweise eine Variationsbreite der Bestrahlungsdauer pro Isoenergieschicht oder der Gesamtbestrahlungsdauer angegeben werden.

Der Effizienzfaktor kann dabei für jeden Spill mit der folgende Gleichung (5) bestimmt werden:

$$EffF_i = \frac{realInt_i}{nomInt_i} \qquad (5)$$

Dabei entspricht $EffF_i$ dem Effizienzfaktor des i-ten Spills. $nomInt_i$ entspricht der nominellen Intensität des Beschleunigers der Partikelbestrahlungsanlage für den i-ten Spill und $realInt_i$ entspricht der realen Intensität des Beschleunigers bei der gewählten nominellen Intensität $nomInt_i$ für den i-ten Spill.

Darüber hinaus kann der Effizienzfaktor EffF auch global gemäß der folgenden Gleichung (6) bestimmt werden:

$$EffF = \frac{1}{numSp} \sum_{i=1}^{numSp} \frac{realInt_i}{nomInt_i} \qquad (6)$$

Wie bei Gleichung (5) entspricht $nomInt_i$ der nominellen Intensität des Beschleunigers für den i-ten Spill und $realInt_i$ entspricht der realen Intensität des Beschleunigers bei der gewählten nominellen Intensität $nomInt_i$ für den i-ten Spill. numSp gibt die Anzahl der Spills an.

Der Effizienzfaktor EffF kann darüber hinaus mit einer der folgenden Gleichungen (7) oder (8) berechnet werden.

$$EffF = \sum_{i=1}^{numSp} \frac{numPartSp_i}{nomInt_i} \times \frac{1}{TSp_i} \times \frac{numPartPlan}{numPartSp_i} \qquad (7)$$

Dabei entspricht numSp wiederum der Anzahl der Spills und $nomInt_i$ der nominalen Intensität des i-ten Spills. $numPartSp_i$ entspricht der Anzahl der Partikel des i-ten Spills, $TSP_i$ der Bestrahlungsdauer des i-ten Spills und numPartPlan der Gesamtanzahl der Partikel gemäß Bestrahlungsplan.

Man erkennt, dass $numPartSp_i$ in Gleichung (7) gekürzt werden kann, wodurch sich Gleichung (8) ergibt:

$$EffF = \sum_{i=1}^{numSp} \frac{numPartPlan}{nomInt_i \times TSp_i} \qquad (8)$$

Erfindungsgemäß kann der Effizienzfaktor auch als Optimierungsparameter eingesetzt werden, wenn dadurch die Gesamtbestrahlungsdauer um mehr als einen vorgegebenen Zeitdauerschwellenwert verkürzt wird.

Der Effizienzfaktor kann beispielsweise durch die Erhöhung des Quellenstroms der Ionenquelle der Partikelbestrahlungsanlage um bis zu 20% erhöht werden. Diese leichte Erhöhung der Effizienz sollte erfindungsgemäß nur dann eingesetzt werden, wenn dadurch eine überproportionale Verringerung der Bestrahlungszeit erzielt werden kann.

Erfindungsgemäß ist es auch möglich, dass für eine Isoenergieschicht eine maximale Bestrahlungsdauer oder eine maximale Anzahl von Spills vorgegeben wird.

Natürlich ist es erfindungsgemäß ebenfalls möglich, für jede Isoenergieschicht eine individuelle oder dieselbe maximale Bestrahlungsdauer oder eine maximale Anzahl von Spills (insbesondere genau ein Spill pro Isoenergieschicht) vorzugeben. Wenn jede Isoenergieschicht mit nur einem Spill bestrahlt wird, kann dies insbesondere bei der Bestrahlung bewegter Objekte zu einer besseren Dosisverteilung führen, da die Rasterpunkte derselben Isoenergieschicht innerhalb einer vergleichsweise kurzen Zeitspanne mit der entsprechenden Energie bzw. Dosis versorgt werden.

Es ist erfindungsgemäß ebenfalls möglich, eine maximale Gesamtbestrahlungsdauer vorzugeben.

Die Vorgabe der maximalen Gesamtbestrahlungsdauer kann zum einen derart umgesetzt werden, dass der erfindungsgemäß bestimmte Bestrahlungsplan sicher eine Gesamtbestrahlungsdauer aufweist, welche nicht länger als die vorgegebene maximale Gesamtbestrahlungsdauer ist. Es ist allerdings ebenfalls möglich, dass die zu optimierende Kostenfunktion die Gesamtbestrahlungsdauer erst dann negativ (d.h. mit einem entsprechenden Zuschlag (Penalty)) berücksichtigt, wenn die Gesamtbestrahlungsdauer länger als die maximale Gesamtbestrahlungsdauer ist. Dabei kann dieser Zuschlag eine nichtlineare Größe aufweisen, was bedeutet, dass der Zuschlag bei einer leichten Überschreitung der maximalen Gesamtbestrahlungsdauer gering ist, aber bei einer größeren Überschreitung nichtlinear mit der Differenz zur maximalen Gesamtbestrahlungsdauer anwächst.

Die negative Berücksichtigung der Überschreitung einer vorgegebenen Begrenzung (beispielsweise der maximalen Bestrahlungsdauer einer Isoenergieschicht oder einer maximalen Anzahl von Spills für eine Isoenergieschicht) in Form eines nichtlinear anwachsenden Zuschlags, welcher dann bei der zu optimierende Kostenfunktion berücksichtigt wird, kann auch bei anderen Ausführungsformen eingesetzt werden.

Im Rahmen der vorliegenden Erfindung wird auch eine Vorrichtung zur Bestimmung eines Bestrahlungsplans für eine Partikelbestrahlungsanlage bereitgestellt. Die Partikelbestrahlungsanlage bestrahlt dabei abhängig von dem bestimmten Bestrahlungsplan ein Zielvolumen innerhalb eines Untersuchungsobjekts mit einem Teilchenstrahl oder Partikelstrahl. Die Vorrichtung umfasst Eingabemittel, Rechenmittel und Ausgabemittel. Mittels der Eingabemittel werden der Vorrichtung das Zielvolumen und eine vorbestimmte Dosisverteilung (Soll-Dosisverteilung) vorgegeben. Die Rechenmittel bestimmen den Bestrahlungsplan derart, dass die Teilchen des Teilchenstrahls entsprechend der vorbestimmten Dosisverteilung in dem Zielvolumen abgegeben werden. Der Bestrahlungsplan wird mit den Ausgabemitteln ausgegeben. Erfindungsgemäß berücksichtigen die Rechenmittel bei der Bestimmung des Bestrahlungsplans eine Bestrahlungsdauer, wobei die Rechenmittel den Bestrahlungsplan derart bestimmen, dass diese Bestrahlungsdauer möglichst kurz ist.

Darüber hinaus stellt die vorliegende Erfindung eine Partikelbestrahlungsanlage mit einer erfindungsgemäßen Vorrichtung bereit.

Die Vorteile der erfindungsgemäßen Vorrichtung und der erfindungsgemäßen Partikelbestrahlungsanlage entsprechen im Wesentlichen den Vorteilen des erfindungsgemäßen Verfahrens, welche vorab im Detail ausgeführt sind, so dass hier auf eine Wiederholung verzichtet wird.

Des Weiteren beschreibt die vorliegende Erfindung ein Computerprogrammprodukt, insbesondere eine Software, welche man in einen Speicher einer programmierbaren Steuereinrichtung bzw. Rechenmitteln einer Partikelbestrahlungsanlage laden kann. Mit diesem Computerprogrammprodukt können alle oder verschiedene vorab beschriebene Ausführungsformen des erfindungsgemäßen Verfahrens ausgeführt werden, wenn das Computerprogrammprodukt in der Steuereinrichtung läuft. Dabei benötigt das Computerprogrammprodukt eventuell Programmmittel, z.B. Bibliotheken und Hilfsfunktionen, um die entsprechenden Ausführungsformen des Verfahrens zu realisieren. Mit anderen Worten soll mit dem auf das Computerprogrammprodukt gerichteten Anspruch insbesondere eine Software unter Schutz gestellt werden, mit welcher eine der oben beschriebenen Ausführungsformen des erfindungsgemäßen Verfahrens ausgeführt werden kann bzw. welche diese Ausführungsform ausführt. Dabei kann es sich bei der Software um einen Quellcode (z.B. C++), der noch compiliert und gebunden oder der nur interpretiert werden muss, oder um einen ausführbaren Softwarecode handeln, der zur Ausführung nur noch in die entsprechenden Rechenmittel bzw. Steuereinrichtung zu laden ist.

Schließlich offenbart die vorliegende Erfindung einen elektronisch lesbaren Datenträger, z.B. eine DVD, ein Magnetband oder einen USB-Stick, auf welchem elektronisch lesbare Steuerinformationen, insbesondere Software (vgl. oben), gespeichert ist. Wenn diese Steuerinformationen (Software) von dem Datenträger gelesen und in Steuermittel bzw. eine Recheneinheit einer Partikelbestrahlungsanlage gespeichert werden, können alle erfindungsgemäßen Ausführungsformen des vorab beschriebenen Verfahrens durchgeführt werden.

Die vorliegende Erfindung ist insbesondere zur Erhöhung des Patientendurchsatzes bei der Partikeltherapie geeignet. Selbstverständlich ist die vorliegende Erfindung nicht auf diesen bevorzugten Anwendungsbereich eingeschränkt, da die vorliegende Erfindung prinzipiell überall dort eingesetzt werden kann, wo mit Teilchen oder Partikeln Energie bzw. eine Dosis in einem Zielvolumen appliziert wird.

Im Folgenden wird die vorliegende Erfindung anhand erfindungsgemäßer Ausführungsformen mit Bezug zu den Figuren im Detail beschrieben.

Fig. 1 stellt schematisch einen Überblick über den Aufbau einer Partikelbestrahlungsanlage dar.

In Fig. 2 ist schematisch dargestellt, wie ein Zielvolumen mittels einer Partikelbestrahlungsanlage bestrahlt wird.

In Fig. 3 ist beispielhaft ein Intensitätsverlauf über der Anzahl der Teilchen für einen Spill dargestellt.

In Fig. 4 ist ein Programmablaufplan eines erfindungsgemäßen Verfahrens dargestellt.

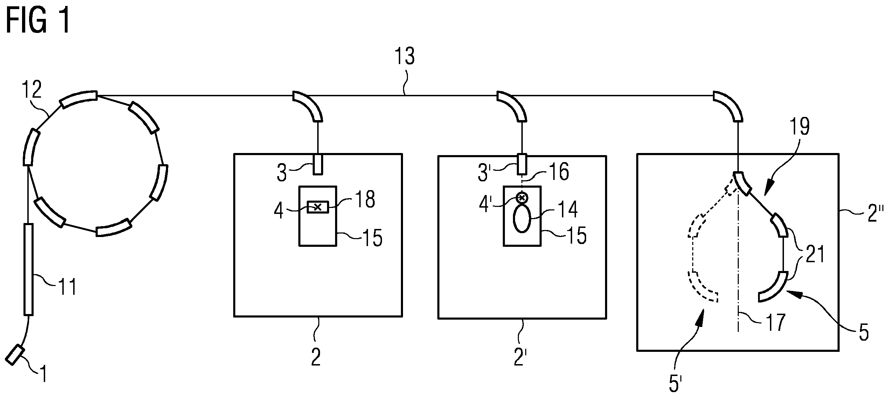

Die in Fig. 1 schematisch dargestellte Partikelbestrahlungsanlage 20 bestrahlt einen auf einer Positionierungsvorrichtung 15 (einem Tisch) liegenden Patienten 14 (siehe Bestrahlungsraum 2') mit einem Partikel bzw. Teilchen 16 umfassenden Strahl, welcher im Folgenden als Partikelstrahl bzw. Teilchenstrahl 16 bezeichnet wird. Mit einem solchen Teilchenstrahl 16 kann beispielsweise ein Tumor des Patienten 14 mit hochenergetischen Partikeln bestrahlt werden. Es ist allerdings auch möglich, die Partikelbestrahlungsanlage 20 zur Bestrahlung eines nicht-lebenden Objektes 18 einzusetzen, wie es im Bestrahlungsraum 2 am Beispiel eines Wasserphantoms 18 dargestellt ist.

Als Partikel bzw. Teilchen werden beispielsweise Protonen, Pionen, Heliumionen, Kohlenstoffionen, aber auch Ionen anderer Elemente eingesetzt. Dazu werden die entsprechenden Partikel in einer Partikelquelle bzw. Ionenquelle 1 erzeugt und in einem Vorbeschleuniger 11 (z. B. einem Linearbeschleuniger) auf ein erstes Energieniveau beschleunigt. Anschließend werden die Teilchen in einem Ringbeschleuniger 12 (z. B. einem Synchrotron oder Zyklotron) auf eine zur Bestrahlung benötigte Energie beschleunigt. Der aus dem Ringbeschleuniger 12 austretende Teilchenstrahl wird von einem Hochenergiestrahl-Transportsystem 13 in einen oder mehrere Bestrahlungsräume 2, 2', 2'' transportiert, und dort zur Bestrahlung eines Zielvolumens eines Patienten 14 eingesetzt. Die Bestrahlung erfolgt von einer festen Richtung aus, so dass der zu bestrahlende Körper 14, 18 vorher raumfest mittels der Positionierungsvorrichtung 15 in dem Bestrahlungsraum 2, 2' angeordnet wird. Die Bestrahlungsräume 2, 2' werden daher auch als so genannte "fixed beam"-Räume bezeichnet. Dagegen existiert im Bestrahlungsraum 2'' eine um eine Achse 17 beweglich angeordnete, insbesondere drehbare Gantry 19, mittels welcher der zu bestrahlende Körper von verschiedenen Richtungen aus bestrahlt werden kann. Dazu wird der Teilchenstrahl 16 mit Hilfe einer Strahlführung 21 der Gantry 19 entsprechend auf den zu bestrahlenden Körper gerichtet. In Fig. 1 sind zwei Positionen 5, 5' dargestellt, obwohl mehrere Positionen möglich sind.

In den Bestrahlungsräumen 2, 2' tritt der Teilchenstrahl 16 aus einem Strahlauslass 3, 3' aus und trifft auf den Körper 14 bzw. 18, in welchem sich das zu bestrahlende Zielvolumen befindet. Das Zielvolumen liegt dabei normalerweise in dem Isozentrum 4, 4' des jeweiligen Bestrahlungsraums 2, 2'.

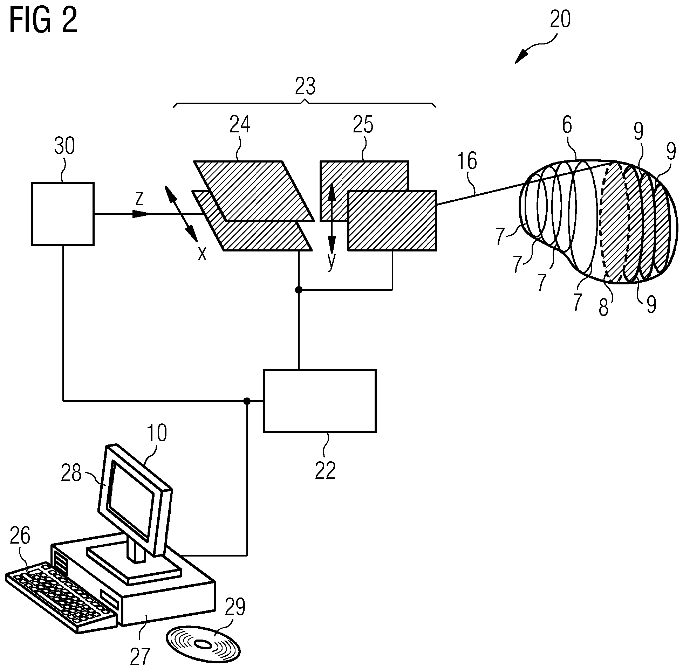

In Fig. 2 ist schematisch ein Zielvolumen 6 dargestellt, welches von einem mittels einer Partikelbestrahlungsanlage 20 erzeugten Teilchenstrahl 16 bestrahlt wird. Die Partikelbestrahlungsanlage 20 umfasst neben einer Bestrahlungsplanungsvorrichtung 10 eine Strahlerzeugungsvorrichtung 30, eine Rasterscan-Einrichtung 23 und eine Steuerung 22 für die Rasterscan-Einrichtung 23. Die Rasterscan-Einrichtung 23 weist wiederum eine erste Partikelablenkung 24 und eine zweite Partikelablenkung 25 auf, welche jeweils insbesondere Magnete umfassen. Mit Hilfe der beiden Partikelablenkungen 24, 25 kann der Teilchenstrahl 16 sowohl horizontal als auch vertikal abgelenkt werden, was durch die zueinander senkrecht stehenden Pfeile x, y dargestellt ist. Daher ist die Rasterscan-Einrichtung 23 in der Lage, den Teilchenstrahl 16 auf einen beliebigen Punkt $(x_i, y_i)$ einer Fläche innerhalb der x-y-Ebene zu lenken. Jeder dieser Punkte wird zusammen mit der jeweils eingesetzten Partikelenergie als Scan Spot, Rasterpunkt oder Abtastpunkt bezeichnet. Demnach ist ein Rasterpunkt zum einen durch die Ausrichtung des Teilchenstrahls 16 (x- bzw. y-Richtung) und zum anderen durch seine Partikelenergie bestimmt. Mit anderen Worten existieren für bestimmte x- und y-Koordinaten mehrere Rasterpunkte mit unterschiedlichen Partikelenergien. Die Partikelenergie bestimmt dabei quasi die Koordinate in der z-Richtung (senkrecht auf der x-bzw. y-Achse), wobei im Allgemeinen gilt, dass die z-Position umso weiter in Richtung des Teilchenstrahls 16 innerhalb des Zielvolumens 6 liegt, umso größer die Partikelenergie ist. Da allerdings die Eindringtiefe vom Gewebe bzw. Material abhängig ist, welches der Teilchenstrahl 16 durchläuft, gilt obiger Zusammenhang nur für dieselben x- bzw. y-Positionen exakt.

Das mit dem Teilchenstrahl 16 zu bestrahlende Zielvolumen 6 wird dabei in Form von Isoenergieschichten 7-9 bestrahlt. In den Rasterpunkten derselben Isoenergieschicht 7-9 werden dabei jeweils Partikel mit derselben Energie appliziert. Unter der Voraussetzung, dass der Teilchenstrahl 16 auf seinem Weg zu der entsprechenden Isoenergieschicht 7-9 ein homogenes Volumen durchläuft, liegen die Isoenergieschichten 7-9 senkrecht zur z-Achse, wie es vereinfachend in Fig. 2 dargestellt ist.

Zur Einstellung des Teilchenstrahls 16 auf eine entsprechende Isoenergieschicht 7-9 wird den Partikeln des Teilchenstrahls 16 jeweils eine entsprechende Anfangsenergie zugewiesen, indem die Partikel auf eine dieser Anfangsenergie entsprechende Geschwindigkeit beschleunigt werden. Die Anfangsenergie beschreibt dabei die Energie eines Partikels, welches dieses Partikel vor dem Auftreffen auf das Objekt 14 bzw. 18 aufweist. Zur Bestrahlung derjenigen Isoenergieschicht 7, welche dem Strahlauslass 3, 3' am nächsten liegt (am weitesten links in Fig. 2), werden dazu Partikel mit der niedrigsten Energie eingesetzt, wohingegen zur Bestrahlung derjenigen Isoenergieschicht 9, welche von dem Strahlauslass 3, 3' am weitesten entfernt angeordnet ist (am weitesten rechts in Fig. 2), die Partikel mit der höchsten Energie verwendet werden.

Zur Bestrahlung des gesamten Zielvolumens 6 werden die Isoenergieschichten 7-9 nacheinander bestrahlt, wobei in der Regel mit der Isoenergieschicht 9, welche am weitesten vom Strahlauslass 3, 3' entfernt ist, begonnen wird und dann mit der jeweils benachbarten Isoenergieschicht fortgefahren wird. Um bestimmte Rasterpunkte innerhalb derselben Isoenergieschicht 7-9 mit unterschiedlichen Energien zu bestrahlen, wird insbesondere die Zeitspanne variiert, während welcher der entsprechende Rasterpunkt von dem Teilchenstrahl 16 bestrahlt wird. Je länger der entsprechende Rasterpunkt von dem Teilchenstrahl 16 bestrahlt wird, desto mehr Energie (höhere Dosis) wird in dem entsprechenden Rasterpunkt deponiert.

Bei dem in Fig. 2 dargestellten Zielvolumen 6 wird aktuell die Isoenergieschicht 8 von dem Teilchenstrahl 16 bestrahlt, während die drei Isoenergieschichten 9 bereits bestrahlt worden sind und die weiter links (in Fig. 2) liegenden vier Isoenergieschichten 7 noch auf ihre Bestrahlung warten.

Bevor das Zielvolumen 6 bestrahlt wird, wird ein Bestrahlungsplan erstellt, mittels welchem das Scannen bzw. Abtasten des Zielvolumens 6 mit dem Teilchenstrahl 16 erfolgt. Der Bestrahlungsplan bestimmt dabei insbesondere Steuerparameter zur Besteuerung der Partikelbestrahlungsanlage 20. Die Erstellung des Bestrahlungsplans wird dabei mit Hilfe einer Bestrahlungsplanungsvorrichtung 10 (beispielsweise einem PC) durchgeführt.

Zur Durchführung der eigentlichen Bestrahlung wird der Bestrahlungsplan von der Bestrahlungsplanungsvorrichtung 10 an die Strahlerzeugungsvorrichtung 30 und die Steuerung 22 der Rasterscan-Einrichtung 23 weitergeleitet. In Fig. 2 ist die Bestrahlungsplanungsvorrichtung 10 quasi als Bestandteil der Partikelbestrahlungsanlage 20 dargestellt. Natürlich ist es ebenso gut erfindungsgemäß möglich, dass der von der Bestrahlungsplanungsvorrichtung 10 erstellte Bestrahlungsplan auf einen Datenträger 29 geladen wird, über welchen dann der Bestrahlungsplan in die Partikelbestrahlungsanlage 20 geladen wird. In diesem Fall müssen die Bestrahlungsplanungsvorrichtung 10 und die Partikelbestrahlungsanlage 20 nicht miteinander kommunikationstechnisch verbunden sein. Darüber hinaus kann zwischen der Erstellung des Bestrahlungsplans und der anhand des Bestrahlungsplans durchgeführten Bestrahlung ein gewisser Zeitraum, beispielsweise mehrere Tage, liegen.

Zur Erstellung des Bestrahlungsplans benötigt die Bestrahlungsplanungsvorrichtung 10 die Lage und die Ausmaße des zu bestrahlenden Zielvolumens 6 (z. B. eines zu bestrahlenden Tumors). Darüber hinaus wird bei der Bestrahlung eines Patienten 14 die Beschaffenheit des Gewebes benötigt, welches von dem Teilchenstrahl 16 auf dem Weg zu dem Zielvolumen 6 durchstrahlt wird. Diese Informationen können beispielsweise mittels eines Computer- oder Magnetresonanztomographen ermittelt werden und dann über entsprechende Eingabemittel 26 an die Bestrahlungsplanungsvorrichtung 10 übermittelt werden. Die Bestrahlungsplanungsvorrichtung 10 bestimmt mit Hilfe ihrer Rechenmittel 27 ausgehend von diesen Informationen und einer vorbestimmten Dosisverteilung (Soll-Dosisverteilung) den Bestrahlungsplan. Der Bestrahlungsplan gibt dabei insbesondere an, wie viele Partikel einer bestimmten Energie an einem Rasterpunkt zu applizieren sind.

Ein Patient muss während der Bestrahlung fixiert werden, um eine Bewegung des Zielvolumens 6 möglichst auszuschließen. Aus diesem Grund sollte die Bestrahlungsdauer möglichst kurz gehalten werden. Darüber hinaus ermöglicht eine kurze Bestrahlungsdauer vorteilhafterweise einen höheren Patientendurchsatz. Auf der anderen Seite sollte die Dosisverteilung gemäß Bestrahlungsplan möglichst gut der Soll-Dosisverteilung entsprechen. Indem erfindungsgemäß bereits bei der Erstellung und Optimierung des Bestrahlungsplans die Bestrahlungsdauer einer Isoenergieschicht oder die Gesamtbestrahlungsdauer minimiert wird, kann ein erfindungsgemäß erstellter Bestrahlungsplan vorteilhafterweise sowohl zu einer kurzen Bestrahlungsdauer als auch zu einer guten Qualität bei der Dosisverteilung führen. Je nach Gewichtung entsprechender Bewertungskriterien (z.B. Optimierungspenalties) kann damit mehr Wert auf die Qualität der Dosisverteilung oder auf die Bestrahlungsdauer gelegt werden.

In Fig. 3 sind für einen Spill für alle von diesem Spill bestrahlten Rasterpunkte die Intensitäten über der Anzahl der Teilchen bzw. Partikelanzahl aufgetragen. Die Partikelanzahl weist zu Beginn des Spills den Wert 0 auf und entspricht anschließend der Summe der Teilchen bzw. Partikel, welche bezüglich des aktuellen Rasterpunkts und zusätzlich bezüglich der zeitlich vorher bestrahlten Rasterpunkte von dem Spill in das Zielvolumen geschossen worden sind. Mit anderen Worten steigt die Partikelanzahl von Rasterpunkt zu Rasterpunkt stetig an. Man erkennt, dass die Intensität zuerst bis zu einer bestimmten Partikelanzahl 31 stark ansteigt und anschließend leicht abfällt. Beispielsweise mit Hilfe der Methode der kleinsten Quadrate kann für den aufsteigenden Ast eine (positive) Steigung m1 und ein y-Achsenabschnitt b1 sowie für den abfallenden Ast eine (negative) Steigung m2 und ein y-Achsenabschnitt b2 bestimmt werden. Ein entsprechender Geradenabschnitt ist in Fig. 3 sowohl für den aufsteigenden als auch für den abfallenden Ast dargestellt.

Indem bei der Bestimmung eines Bestrahlungsplans die von einem Spill zu erzeugende Intensität anhand der in Fig. 3 dargestellten Geradenabschnitte berechnet wird, stimmt die bei der Bestimmung des Bestrahlungsplans berücksichtigte Intensität besser mit der später applizierten Intensität überein, als dies nach dem Stand der Technik der Fall ist. Dadurch kann vorteilhafterweise auch die Bestrahlungsdauer eines Spills bzw. einer Isoenergieschicht und damit auch die gesamte Bestrahlungsdauer gemäß Bestrahlungsplan genauer bestimmt werden. Dies führt wiederum dazu, dass die Auswirkungen erfindungsgemäßer Maßnahmen zur Verringerung der Bestrahlungszeit auf die Bestrahlungszeit genauer kalkuliert werden können, so dass zumindest mit größerer Sicherheit (d.h. mit einer geringeren Abweichung zum tatsächlichen Ergebnis) ein hinsichtlich der Bestrahlungszeit optimierter Bestrahlungsplan erstellt werden kann.

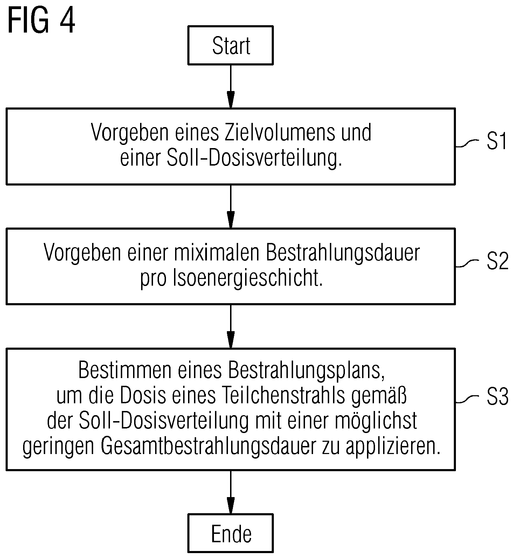

In Fig. 4 ist ein erfindungsgemäßes Verfahren in Form eines Flussablaufplans dargestellt.

Im ersten Schritt S1 wird das Zielvolumen und eine Soll-Dosisverteilung bzw. Zieldosisverteilung vorgegeben. In einem folgenden zweiten Schritt S2 wird pro Isoenergieschicht eine maximale Bestrahlungsdauer vorgegeben, welche insbesondere vorgibt, wie lange die jeweilige Isoenergieschicht maximal mit einem Teilchenstrahl bestrahlt werden darf.

Im anschließenden dritten Verfahrensschritt S3 wird der Bestrahlungsplan bestimmt, um die Energie des Teilchenstrahls gemäß der Soll-Dosisverteilung mit einer möglichst kurzen Gesamtbestrahlungsdauer aufzubringen, wobei die pro Isoenergieschicht vorgegebene maximale Bestrahlungsdauer nicht überschritten werden Die Ausführungsbeispiele, die nicht unter den Schutzumfang der beigefügten Ansprüche fallen, dienen lediglich zu illustrativen Zwecken und sind nicht Gegenstand der gegenwärtigen Erfindung. Die Erfindung wird durch die folgenden Ansprüche definiert.

## Patentansprüche

1.  Verfahren zum Bestimmen eines Bestrahlungsplans für eine Partikelbestrahlungsanlage (20),
    wobei der Bestrahlungsplan zur Planung der Bestrahlung eines Zielvolumens (6) innerhalb eines Untersuchungsobjekts (14; 18) mit einem Teilchenstrahl (16) mit der Partikelbestrahlungsanlage (20) vorgesehen ist,
    wobei das Zielvolumen (6) und eine vorbestimmte Dosisverteilung vorgegeben werden,
    wobei der Bestrahlungsplan bestimmt wird, um die Energie des Teilchenstrahls (16) gemäß der vorbestimmten Dosisverteilung in dem Zielvolumen (6) zu applizieren,
    **dadurch gekennzeichnet,**
    **dass** der Bestrahlungsplan anhand eines Optimierungsverfahrens bestimmt wird,
    **dass** das Optimierungsverfahren eine Maßzahl abhängig von einem Unterschied zwischen der vorbestimmten Dosisverteilung und einer Dosisverteilung gemäß Bestrahlungsplan und abhängig von der Bestrahlungsdauer bestimmt,
    **dass** das Optimierungsverfahren den Bestrahlungsplan mit der besten Maßzahl bestimmt, und
    **dass** die Maßzahl umso besser ist, je besser die vorbestimmte Dosisverteilung der Dosisverteilung gemäß Bestrahlungsplan entspricht und je kürzer die Bestrahlungsdauer ist.

2.  Verfahren nach Anspruch 1,
    **dadurch gekennzeichnet,**
    **dass** die Bestrahlungsdauer einer Gesamtbestrahlungsdauer des Bestrahlungsplans oder einer Bestrahlungsdauer einer Isoenergieschicht (7-9) im Zielvolumen (6) entspricht.

3.  Verfahren nach Anspruch 1 oder 2,
    **dadurch gekennzeichnet,**
    **dass** die Gesamtbestrahlungsdauer T wie folgt bestimmt wird

$$T = numSp \times TBschl + \sum_{i=1}^{numSp} TSp_i \, ,$$

wobei numSp der Anzahl der Spills entspricht,
wobei TBschl einer Zeitspanne entspricht, in welcher die Partikel eines neuen Spills beschleunigt werden, und
wobei $TSp_i$ der Bestrahlungsdauer des i-ten Spills entspricht, und

wobei die Bestrahlungsdauer $TSp_i$ des i-ten Spills wie folgt bestimmt wird

$$TSp_i = \frac{numPartSp_i}{nomInt_i \times EffF}$$

wobei $numPartSp_i$ der Anzahl der Partikel im i-ten Spill entspricht,
wobei $nomInt_i$ der nominalen Intensität des i-ten Spills entspricht, und
wobei $EffF$ einem Effizienzfaktor der Partikelbestrahlungsanlage (20) entspricht.

4. Verfahren nach Anspruch 3,
**dadurch gekennzeichnet,**
**dass** die Bestrahlungsdauer $TSp_i$ des i-ten Spills wie folgt bestimmt wird

$$TSp_i = \sum_{j=1}^{numSpot_i} \frac{numPartSpot_{j,i}}{Int_{j,i}} \, ,$$

wobei $numSpot_i$ der Anzahl der Spots des i-ten Spills entspricht,
wobei $numPartSpot_{j,i}$ der Anzahl der Partikel des j-ten Spots im i-ten Spill entspricht,
wobei $Int_{j,i}$ der Intensität während der Bestrahlung des j-ten Spots im i-ten Spill entspricht,
wobei die Intensität $Int_{j,i}$ wie folgt bestimmt wird

$$Int_{j,i} = m \times \sum_{k=1}^{j} numPartSpot_{k,i} + b \, ,$$

wobei m einer Steigung ($m_1$; $m_2$) einer Geraden und b einem y-Achsenabschnitt ($b_1$; $b_2$) der Geraden entspricht, und
wobei die Gerade einen empirisch ermittelten Verlauf der Intensität eines Spills über der Partikelanzahl beschreibt.

5. Verfahren nach Anspruch 4,
**dadurch gekennzeichnet,**
**dass** die Steigung m einen ersten Steigungswert ($m_1$) und der y-Achsenabschnitt einen ersten Abschnittswert ($b_1$) aufweist, wenn die Partikelanzahl kleiner als ein Partikelanzahlschwellenwert (31) ist,
**dass** die Steigung m einen zweiten Steigungswert ($m_2$) und der y-Achsenabschnitt einen zweiten Abschnittswert ($b_2$) aufweist, wenn die Partikelanzahl größer als der Partikelanzahlschwellenwert (31) ist,
**dass** der erste Steigungswert ($m_1$) positiv und der zweite Steigungswert ($m_2$) negativ ist, und
**dass** der zweite Abschnittswert ($b_2$) linear von dem Effizienzfaktor abhängt.

6. Verfahren nach einem der vorhergehenden Ansprüche,
**dadurch gekennzeichnet,**
**dass** der Bestrahlungsplan abhängig von einem Effizienzfaktor der Partikelbestrahlungsanlage (20) bestimmt wird.

7. Verfahren nach Anspruch 6,
**dadurch gekennzeichnet,**
**dass** der Effizienzfaktor $EffF_i$ für den i-ten Spill wie folgt bestimmt wird

$$EffF_i = \frac{realInt_i}{nomInt_i} \, ,$$

wobei $realInt_i$ der realen Intensität der Partikelbestrahlungsanlage (20) für den i-ten Spill entspricht, und

wobei $nomInt_i$ der nominellen Intensität der Partikelbestrahlungsanlage (20) für den i-ten Spill entspricht.

**8.** Verfahren nach Anspruch 6,
**dadurch gekennzeichnet,**
**dass** der Effizienzfaktor EffF wie folgt bestimmt wird

$$EffF = \frac{1}{numSp} \sum_{i=1}^{numSp} \frac{realInt_i}{nomInt_i},$$

wobei numSp der Anzahl der Spills entspricht,
wobei $realInt_i$ der realen Intensität der Partikelbestrahlungsanlage (20) entspricht, und wobei $nomInt_i$ der nominellen Intensität der Partikelbestrahlungsanlage (20) entspricht.

**9.** Verfahren nach Anspruch 6,
**dadurch gekennzeichnet,**
**dass** der Effizienzfaktor EffF wie folgt bestimmt wird

$$EffF = \sum_{i=1}^{numSp} \frac{numPartPlan}{nomInt_i \times TSp_i},$$

wobei numSp der Anzahl der Spills entspricht,
wobei $nomInt_i$ der nominalen Intensität des i-ten Spills entspricht,
wobei $TSp_i$ der Bestrahlungsdauer des i-ten Spills entspricht, und
wobei numPartPlan der Gesamtanzahl der Partikel gemäß dem Bestrahlungsplan entspricht.

**10.** Verfahren nach einem der Ansprüche 6 bis 9,
**dadurch gekennzeichnet,**
**dass** der Effizienzfaktor verbessert wird, wenn dadurch die Gesamtbestrahlungsdauer um mehr als einen vorgegebenen Zeitdauerschwellenwert verkürzt wird.

**11.** Verfahren nach einem der vorhergehenden Ansprüche,
**dadurch gekennzeichnet,**
**dass** eine maximale Bestrahlungsdauer für eine Isoenergieschicht (7-9) vorgegeben wird, oder
**dass** eine maximale Anzahl von Spills für eine Isoenergieschicht (7-9) vorgegeben wird.

**12.** Verfahren nach einem der vorhergehenden Ansprüche,
**dadurch gekennzeichnet,**
**dass** eine maximale Gesamtbestrahlungsdauer vorgegeben wird.

**13.** Vorrichtung zum Bestimmen eines Bestrahlungsplans für eine Partikelbestrahlungsanlage (20),
wobei der Bestrahlungsplan zur Planung der Bestrahlung eines Zielvolumens (6) innerhalb eines Untersuchungsobjekts (14; 18) mit einem Teilchenstrahl (16) mit der Partikelbestrahlungsanlage (20) vorgesehen ist,
wobei die Vorrichtung (10) Eingabemittel (26), Rechenmittel (27) und Ausgabemittel (28) umfasst,
wobei der Vorrichtung (10) mittels der Eingabemittel (26) das Zielvolumen (6) und eine vorbestimmte Dosisverteilung vorgebbar sind,
wobei die Rechenmittel (27) den Bestrahlungsplan bestimmen, um die Energie des Teilchenstrahls (16) gemäß der vorbestimmten Dosisverteilung in dem Zielvolumen (6) zu applizieren, wobei die Ausgabemittel (28) den Bestrahlungsplan ausgeben,
**dadurch gekennzeichnet,**
**dass** die Rechenmittel (27) bei der Bestimmung des Bestrahlungsplans ein Optimierungsverfahren anwenden, bei dem eine Maßzahl abhängig von einem Unterschied zwischen der vorbestimmten Dosisverteilung und einer Dosisverteilung gemäß Bestrahlungsplan und abhängig von der Bestrahlungsdauer bestimmt wird, und bei dem die Maßzahl umso besser ist, je besser die vorbestimmte Dosisverteilung der Dosisverteilung gemäß Bestrahlungsplan entspricht und je kürzer die Bestrahlungsdauer ist.

**14.** Vorrichtung nach Anspruch 13,
**dadurch gekennzeichnet,**
**dass** die Vorrichtung (10) zur Durchführung des Verfahrens nach einem der Ansprüche 1 bis 12 ausgestaltet ist.

**15.** Partikelbestrahlungsanlage mit einer Vorrichtung (10) nach Anspruch 13 oder 14.

**16.** Computerprogrammprodukt, welches ein Programm umfasst und direkt in einen Speicher einer programmierbaren Steuereinrichtung einer Partikelbestrahlungsanlage (20) ladbar ist, mit Programm-Mitteln, um alle Schritte des Verfahrens nach einem der Ansprüche 1 bis 12 auszuführen, wenn das Programm in der Steuereinrichtung der Partikelbestrahlungsanlage (20) ausgeführt wird.

**17.** Elektronisch lesbarer Datenträger mit darauf gespeicherten elektronisch lesbaren Steuerinformationen, welche derart ausgestaltet sind, dass sie bei Verwendung des Datenträgers (29) in einer Steuereinrichtung einer Partikelbestrahlungsanlage (20) das Verfahren nach einem der Ansprüche 1 bis 12 durchführen.

**Claims**

**1.** Method for determining an irradiation plan for a particle irradiation system (20),
wherein the irradiation plan is provided for planning the irradiation of a target volume (6) within a test object (14; 18) with the aid of a particle beam (16) using the particle irradiation system (20),
wherein the target volume (6) and a predetermined dose distribution are prescribed, and
wherein the irradiation plan is determined in order to apply the energy of the particle beam (16) in accordance with the predetermined dose distribution in the target volume (6),
**characterized**
**in that** the irradiation plan is determined with the aid of an optimization method,
**in that** the optimization method determines a measure as a function of a difference between the predetermined dose distribution and a dose distribution according to the irradiation plan and as a function of the irradiation time,
**in that** the optimization method determines the irradiation plan with the aid of the best measure, and
**in that** the measure is better, the better the predetermined dose distribution corresponds to the dose distribution according to the irradiation plan, and the shorter the irradiation time.

**2.** Method according to Claim 1,
**characterized**
**in that** the irradiation time corresponds to a total irradiation time of the irradiation plan or to an irradiation time of an isoenergy layer (7-9) in the target volume (6).

**3.** Method according to Claim 1 or 2,
**characterized**
**in that** the total irradiation time T is determined as follows

$$T = numSp \times TBschl + \sum_{i=1}^{numSp} TSp_i \, ,$$

wherein numSp corresponds to the number of spills,
wherein TBschl corresponds to a time interval in which the particles of a new spill are accelerated, and
wherein $TSp_i$ corresponds to the irradiation time of the ith spill, and
wherein the irradiation time $TSp_i$ of the ith spill is determined as follows

$$TSp_i = \frac{numPartSp_i}{nomInt_i \times EffF}$$

wherein $numPartSp_i$ corresponds to the number of the particles in the ith spill,
wherein $nomInt_i$ corresponds to the nominal intensity of the ith spill, and

wherein EffF corresponds to an efficiency factor of the particle irradiation system (20).

4. Method according to Claim 3,
**characterized**
**in that** the irradiation time $TSp_i$ of the ith spill is determined as follows

$$TSp_i = \sum_{j=1}^{numSpot_i} \frac{numPartSpot_{j,i}}{Int_{j,i}} \, ,$$

wherein numSpoti corresponds to the number of the spots of the ith spill,
wherein $numPartSpot_{j,i}$ corresponds to the number of the particles of the jth spot in the ith spill,
wherein $Int_{j,i}$ corresponds to the intensity during the irradiation of the jth spot in the ith spill,
wherein the intensity $Int_{j,i}$ is determined as follows

$$Int_{j,i} = m \times \sum_{k=1}^{j} numPartSpot_{k,i} + b \, ,$$

wherein m corresponds to a slope ($m_1$; $m_2$) of a straight line, and b corresponds to a y-axis intercept ($b_1$; $b_2$) of the straight line, and
wherein the straight line describes an empirically determined profile of the intensity of a spill against the particle number.

5. Method according to Claim 4,
**characterized**
**in that** the slope m has a first slope value ($m_1$), and the y-axis intercept has a first intercept value ($b_1$) when the number of particles is less than a threshold value (31) for the number of particles,
**in that** the slope m has a second slope value ($m_2$) and the y-axis intercept has a second intercept value ($b_2$) when the number of particles is greater than the threshold value (31) for the number of particles,
**in that** the first slope value ($m_1$) is positive and the second slope value ($m_2$) is negative, and
**in that** the second intercept value ($b_2$) is a linear function of the efficiency factor.

6. Method according to one of the preceding claims,
**characterized**
**in that** the irradiation plan is determined as a function of an efficiency factor of the particle irradiation system (20).

7. Method according to Claim 6,
**characterized**
**in that** the efficiency factor $EffF_i$ for the ith spill is determined as follows

$$EffF_i = \frac{realInt_i}{nomInt_i} \, ,$$

wherein reallnti corresponds to the real intensity of the particle irradiation system (20) for the ith spill, and wherein nomlnti corresponds to the nominal intensity of the particle irradiation system (20) for the ith spill.

8. Method according to Claim 6,
**characterized**
**in that** the efficiency factor EffF is determined as follows

$$EffF = \frac{1}{numSp} \sum_{i=1}^{numSp} \frac{realInt_i}{nomInt_i} ,$$

wherein numSp corresponds to the number of spills,
wherein $realInt_i$ corresponds to the real intensity of the particle irradiation system (20), and
wherein nomInti corresponds to the nominal intensity of the particle irradiation system (20).

9. Method according to Claim 6,
**characterized**
**in that** the efficiency factor EffF is determined as follows

$$EffF = \sum_{i=1}^{numSp} \frac{numPartPlan}{nomInt_i \times TSp_i} ,$$

wherein numSp corresponds to the number of spills,
wherein nomInti corresponds to the nominal intensity of the ith spill,
wherein $TSp_i$ corresponds to the irradiation time of the ith spill, and
wherein numPartPlan corresponds to the total number of the particles in accordance with the irradiation plan.

10. Method according to one of Claims 6 to 9,
**characterized**
**in that** the efficiency factor is improved when the total irradiation time is shortened thereby by more than a prescribed timing threshold.

11. Method according to one of the preceding claims,
**characterized**
**in that** a maximum irradiation time is prescribed for an isoenergy layer (7-9), or
**in that** a maximum number of spills is prescribed for an isoenergy layer (7-9).

12. Method according to one of the preceding claims,
**characterized**
**in that** a maximum total irradiation time is prescribed.

13. Device for determining an irradiation plan for a particle irradiation system (20),
wherein the irradiation plan is provided for planning the irradiation of a target volume (6) within a test object (14; 18) with the aid of a particle beam (16), using the particle irradiation system (20),
wherein the device (10) comprises input means (26), computing means (27) and output means (28),
wherein the target volume (6) and a predetermined dose distribution can be prescribed for the device (10) by means of the input means (26),
wherein the computing means (27) determine the irradiation plan in order to apply the energy of the particle beam (16) in accordance with the predetermined dose distribution in the target volume (6),
wherein the output means (28) output the irradiation plan,
**characterized**
**in that** the computing means (27) apply an optimization method when determining the irradiation plan, in which a measure is determined as a function of a difference between the predetermined dose distribution and a dose distribution according to the irradiation plan and as a function of the irradiation time, and in which the measure is better, the better the predetermined dose distribution corresponds to the dose distribution according to the irradiation plan, and the shorter the irradiation time.

14. Device according to Claim 13,
**characterized**
**in that** the device (10) is configured to carry out the method according to one of Claims 1 to 12.

15. Particle irradiation system having a device (10) according to Claim 13 or 14.

**16.** Computer program product which comprises a program and can be loaded directly into a memory of a programmable controller of a particle irradiation system (20), having program means designed to carry out all steps of the method according to one of claims 1 to 12 when the program is executed in the controller of the particle irradiation system (20).

**17.** Electronically readable data carrier on which there is stored electronically readable control information which is configured in such a way that when the data carrier (29) is used in a controller of a particle irradiation system (20) said information carries out the method according to one of Claims 1 to 12.

## Revendications

**1.** Procédé de détermination d'un plan d'exposition d'une installation (20) d'exposition à des particules, dans lequel le plan d'exposition est prévu pour planifier l'exposition d'un volume (6) cible au sein d'un objet (14 ; 18) à examiner à un faisceau (16) de particules par l'installation (20) d'exposition à des particules, dans lequel on prescrit le volume (6) cible et une répartition de dose déterminée à l'avance, dans lequel on détermine le plan d'exposition pour appliquer au volume (6) cible l'énergie du faisceau (16) de particules suivant la répartition de dose déterminée à l'avance, **caractérisé**
**en ce que** l'on détermine le plan d'exposition à l'aide d'un procédé d'optimisation,
**en ce que** le procédé d'optimisation détermine un indice en fonction d'une différence entre la répartition de dose déterminée à l'avance et une répartition de dose suivant un plan d'exposition et en fonction de la durée d'exposition,
**en ce que** le procédé d'optimisation détermine le plan d'exposition ayant l'indice le meilleur, et
**en ce que** l'indice est d'autant meilleur que la répartition de dose déterminée à l'avance correspond à la répartition de dose suivant le plan d'exposition et que la durée d'exposition est plus courte.

**2.** Procédé suivant la revendication 1,
**caractérisé**
**en ce que** la durée d'exposition correspond à une durée d'exposition totale du plan d'exposition ou à une durée d'exposition d'une couche (7 à 9) d'iso-énergie du volume (6) cible.

**3.** Procédé suivant la revendication 1 ou 2,
**caractérisé**
**en ce que** l'on détermine la durée T d'exposition totale de la manière suivante

$$T = numSp \ x \ TBschl + \sum_{I=1}^{numSp} TSp_i$$

dans laquelle numSp correspond au nombre des spills,
dans laquelle TBscl correspond à un laps de temps, dans lequel les particules d'un nouveau spill sont accélérées, et
dans laquelle $TSp_i$ correspond à la durée d'exposition du ième spills, et
dans laquelle on détermine la durée $TSp_i$ d'exposition du ième spills de la manière suivant

$$TSp_i = \frac{numPartSp_i}{nomInt_i \ x \ EffF}$$

dans laquelle $numPartSp_i$ correspond au nombre des particules dans le ième spill,
dans laquelle $nomInt_i$ correspondant à l'intensité nominale du ième spill, et
dans laquelle EffF correspondant à un facteur d'efficacité de l'installation (20) d'exposition à des particules.

**4.** Procédé suivant la revendication 3,
**caractérisé**
**en ce que** l'on détermine la durée $TSp_i$ d'exposition du ième spill de la manière suivante

$$TSp_i = \sum_{j=1}^{numSpot_i} \frac{numPartSpot_{j,i}}{Int_{j,i}}$$

dans laquelle numSpoti correspond au nombre des spots du ième spill,

dans laquelle numPartSpot$_{j,i}$ correspond au nombre des particules du jème spots dans le ième spill,

dans laquelle Int$_{j,i}$ correspond à l'intensité pendant l'exposition du jème spots dans le ième spill,

dans laquelle on détermine l'intensité Int$_{j,i}$ de la manière suivante

$$Int_{j,i} = m \; x \sum_{k=1}^{j} numPartSpot_{k,i} + b$$

dans laquelle m correspond à une pente (m$_1$ ; m$_2$) d'une droite et b à une partie (b$_1$ ; b$_2$) d'axe y de la droite, et dans laquelle la droite décrit une courbe déterminée empiriquement de l'intensité d'un spill sur le nombre de particules.

5. Procédé suivant la revendication 4,
   **caractérisé**
   **en ce que** la pente m a une première valeur (m$_1$) de pente et le segment d'axe y une première valeur (b$_1$) de segment, si le nombre de particules est plus petit qu'une valeur (31) de seuil de nombre de particules,
   **en ce que** la pente m a une deuxième valeur (m$_2$) de pente et le segment d'axe y une deuxième valeur (b$_2$) de segment, si le nombre de particules est plus grand que la valeur (31) de seuil de nombre de particules,
   **en ce que** la première valeur (m$_1$) de pente est positive et la deux valeur (m$_2$) de pente est négative, et
   **en ce que** la deuxième valeur (b$_2$) de segment dépend linéairement du facteur d'efficacité.

6. Procédé suivant l'une des revendications précédentes,
   **caractérisé**
   **en ce que** l'on détermine le plan d'exposition en fonction d'un facteur d'efficacité de l'installation (20) d'exposition à des particules.

7. Procédé suivant la revendication 6,
   **caractérisé**
   **en ce que** l'on détermine le facteur EffF$_i$ d'efficacité pour le ième spill de la manière suivante

$$EffF_i = \frac{realInt_i}{nomInt_i}$$

dans laquelle realInt$_i$ correspond à l'intensité réelle de l'installation (20) d'exposition à des particules pour ième spill, et

dans laquelle nomInt$_i$ correspond à l'intensité nominale de l'installation (20) d'exposition à des particules pour le ième spill.

8. Procédé suivant la revendication 6,
   **caractérisé en ce que** l'on détermine le facteur EffF d'efficacité de la manière suivante

$$EffF = \frac{1}{numSp} \sum_{i=1}^{numSp} \frac{realInt_i}{nomInt_i}$$

dans laquelle numSp correspond au nombre de spills,

dans laquelle realInt$_i$ correspond à l'intensité réelle de l'installation (20) d'exposition à des particules, et

dans laquelle nomInt$_i$ correspond à l'intensité nominale de l'installation (20) d'exposition à des particules.

**9.** Procédé suivant la revendication 6,
**caractérisé**
**en ce que** l'on détermine le facteur EffF d'efficacité de la manière suivante

$$EffF = \sum_{i=1}^{numSp} \frac{numPartPlan}{nomInt_i \; x \; TSp_i}$$

dans laquelle numSp correspond au nombre de spills,
dans laquelle $nomInt_i$ correspond à l'intensité nominale du ième spill,
dans lequel $TSp_i$ correspond à la durée d'exposition du ième spill, et
dans laquelle numPartPlan correspond au nombre total des particules suivant le plan d'exposition.

**10.** Procédé suivant l'une des revendications 6 à 9,
caractérisé^
en ce que l'on améliore le facteur d'efficacité si, ainsi, on écourte la durée d'exposition totale de plus d'une valeur de seuil de durée donnée à l'avance.

**11.** Procédé suivant l'une des revendications précédentes,
**caractérisé**
**en ce que** l'on prescrit une durée d'exposition maximum d'une couche (7 à 9) d'iso-énergie, ou
**en ce que** l'on prescrit un nombre maximum de spills d'une couche (7 à 9) d'iso-énergie.

**12.** Procédé suivant l'une des revendications précédentes,
**caractérisé**
**en ce que** l'on prescrit une durée d'exposition totale maximum.

**13.** Dispositif de détermination d'un plan d'exposition d'une installation (20) d'exposition à des particules,
dans lequel le plan d'exposition est prévu pour planifier l'exposition d'un volume (6) cible au sein d'un objet (14 ; 18) à examiner à un faisceau (16) de particules par l'installation (20) d'exposition à des particules,
dans lequel le dispositif (10) comprend des moyens (26) d'entrée, des moyens (27) de calcul et des moyens (28) de sortie,
dans lequel il peut être prescrit au dispositif (10), à l'aide des moyens (26) d'entrée, le volume (6) cible et une répartition de dose déterminée à l'avance,
dans lequel les moyens (27) de calcul déterminent le plan d'exposition pour appliquer au volume (6) cible l'énergie du faisceau (16) de particules suivant la répartition de dose déterminée à l'avance, les moyens (28) de sortie donnant le plan d'exposition,
**caractérisé**
**en ce que** les moyens (27) de calcul appliquent, lors de la détermination du plan d'exposition, un procédé d'optimisation, dans lequel on détermine un indice en fonction des différences entre la répartition de dose déterminée à l'avance et une répartition de dose suivant un plan d'exposition et en fonction de la durée d'exposition et dans lequel l'indice est d'autant meilleur que la répartition de dose déterminée à l'avance correspond mieux à la répartition de dose suivant le plan d'exposition et que la durée d'exposition est plus courte.

**14.** Dispositif suivant la revendication 13,
**caractérisé**
**en ce que** le dispositif (10) est conformé pour effectuer le procédé suivant l'une des revendications 1 à 12.

**15.** Installation d'exposition des particules, comprenant un dispositif (10) suivant la revendication 13 ou 14.

**16.** Produit de programme d'ordinateur, qui comprend un programme et qui peut être chargé directement dans la mémoire d'un dispositif de commande programmable d'une installation (20) d'exposition à des particules, comprenant des moyens de programme pour effectuer tous les stades du procédé suivant l'une des revendications 1 à 12, lorsque le programme est réalisé dans le dispositif de commande de l'installation (20) d'exposition à des particules.

**17.** Support de données déchiffrables électroniquement, sur lequel sont mémorisées des informations de commande déchiffrables électroniquement, et conformé de manière à ce que, lorsque l'on utilise le support (29) de données dans le dispositif de commande d'une installation (20) d'exposition à des particules, elles effectuent le procédé suivant l'une des revendications 1 à 12.

# FIG 1

EP 2 814 572 B1

EP 2 814 572 B1

FIG 2

19

FIG 3

EP 2 814 572 B1

# FIG 4

Start

Vorgeben eines Zielvolumens und
einer Soll-Dosisverteilung.  S1

Vorgeben einer miximalen Bestrahlungsdauer
pro Isoenergieschicht.  S2

Bestimmen eines Bestrahlungsplans,
um die Dosis eines Teilchenstrahls gemäß
der Soll-Dosisverteilung mit einer möglichst
geringen Gesamtbestrahlungsdauer zu applizieren.  S3

Ende